# EUROPEAN PATENT APPLICATION

(11) **EP 4 534 676 A1**
(43) Date of publication of application: **09.04.2025**
(21) Application number: 23816087.3
(22) Date of filing: 31.05.2023
(51) Int. Cl.: C12N 15/62, A61K 35/12, A61K 35/17, A61K 48/00, A61P 1/00, A61P 1/04, A61P 1/16, A61P 1/18, A61P 11/00, A61P 15/00, A61P 17/00, A61P 25/00, A61P 35/00, A61P 43/00, C12N 1/15, C12N 1/19, C12N 1/21, C12N 5/10, C12N 15/63, C12P 21/02

(54) **CHIMERIC ANTIGEN RECEPTOR-EXPRESSING CELL THAT TARGETS EGFR**

(30) Priority: 31.05.2022 JP 2022088480
(71) Applicant: Shinshu University, Matsumoto-shi, Nagano 390-8621 (JP); KYOTO PREFECTURAL PUBLIC UNIVERSITY CORPORATION, Kyoto-shi, Kyoto 602-8566 (JP)
(72) Inventor: NAKAZAWA, Yozo, Matsumoto-shi, Nagano 390-8621 (JP); HIRABAYASHI, Koichi, Matsumoto-shi, Nagano 390-8621 (JP); CHINSUWAN, Thanyavi, Matsumoto-shi, Nagano 390-8621 (JP); HASEGAWA, Aiko, Matsumoto-shi, Nagano 390-8621 (JP); YAGYU, Shigeki, Kyoto-shi, Kyoto 602-8566 (JP)
(74) Representative: Vossius & Partner Patentanwälte Rechtsanwälte mbB
(86) International application number: PCT/JP2023/020214
(87) International publication number: WO 2023/234330

(57) **Abstract**

An object of the present invention is to provide EGFR CAR-T cells expected to be effective for tumors expressing EGFR. The present invention provides a polynucleotide encoding a chimeric antigen receptor (CAR) protein having a target-binding domain that binds to epidermal growth factor receptor (EGFR), a transmembrane domain, and an intracellular signaling domain, wherein the target-binding domain is a ligand for EGFR, and a vector comprising the polypeptide, and a genetically modified cell having the polypeptide or the vector introduced thereinto.

## Description

### Technical Field

The present invention relates to a polynucleotide encoding a chimeric antigen receptor and a vector comprising the polynucleotide useful in the field of adoptive immunotherapy, and a genetically modified cell expressing the chimeric antigen receptor and a method for producing the genetically modified cell.

### Background Art

Epidermal growth factor receptor (EGFR) is a transmembrane tyrosine kinase receptor, and composed of an extracellular region, a transmembrane region, and a cytoplasmic region. EGFR binds to its ligand such as EGF, TGF-α, and is involved in control of cell growth, survival, differentiation, and transfer via Ras/Raf/MEK/ERK, PI3K/Akt/mTOR, JAK/STAT, and PKC signaling pathways. Overexpression of EGFR and dysregulation of EGFR signals due to gene mutation cause formation of tumor, in particular, epithelial malignant tumor.

High expression of EGFR has been found in a wide range of cancer species, and EGFR is believed to be an ideal target of cancer treatment. Antibody drugs and molecular target drugs targeting EGFR have already been put on the market, and such drugs have been incorporated in standard medical therapy for some tumors. However, anti-EGFR antibodies are disadvantageously less effective for RAS gene mutation-positive groups, and EGFR tyrosine kinase inhibitors disadvantageously cause tolerization with high frequency even if they produce a response once. Accordingly, appearance of a new modality targeting EGFR is desired.

Chimeric antigen receptor (CAR)-T cells have been expected as a promising next-generation modality for intractable cancers in recent years. In relation to this, Non Patent Literature 1 has reported examination of antitumor effects of scFv-type CAR-T cells comprising a single-chain antibody (scFv) derived from an anti-EGFR antibody as a target-binding domain in xenograft mouse models. Non Patent Literatures 2 and 3 have reported that phase I clinical trial for scFv-type CAR-T cells targeting EGFR was carried out for non-small cell lung cancer and pancreatic cancer patients with safety. However, the effects of such scFv-type CAR-T cells targeting EGFR are limited.

On the other hand, as a method for producing CAR-T cells targeting a receptor, the present inventors have reported design techniques for CAR comprising a receptor ligand instead of a conventional scFV as the target-binding domain of CAR (ligand-type CAR) (Non Patent Literature 4, Patent Literature 1). In addition, the present inventors have reported that the effects and safety of the ligand-type CAR can be enhanced through optimization of the ligand (such as production of a ligand mutant) (Non Patent Literature 5).

### Citation List

### Patent Literature

### Patent Literature 1: WO 2020/085480

### Non Patent Literature

Non Patent Literature 1: Lin X et al., Clinical & Translational Immunology, 2020, 9(5): e1135
Non Patent Literature 2: Liu Y et al., Cytotherapy, 2020, 22(10): 573-580
Non Patent Literature 3: Zhang Y et al., J Cancer Res Clin Oncol., 2021, 147(12): 3725-3734
Non Patent Literature 4: Nakazawa Y et al., J Hematol Oncol., 2016, 9: 27
Non Patent Literature 5: Hasegawa A et al., Clin Transl Immunology., 2021, 10(5): e1282

### Summary of Invention

### Technical Problem

As described above, even clinical trials have been carried out for scFv-type CAR-T cells targeting EGFR, and the safety has been demonstrated; however, no sufficient efficacy has been demonstrated. Thus, there is demand for development of novel EGFR CAR-T cells expected to be more effective for tumors expressing EGFR.

### Solution to Problem

The present inventors have found for the first time that, when introducing a gene of CAR comprising a ligand for EGFR as a target-binding domain into cells, the CAR expression is maintained over time at high levels, and that the resulting ligand-type EGFR CAR-T cells exhibit remarkable antitumor effects on EGFR-expressing cancer cells.

Specifically, the present invention includes the followings.
[1] A polynucleotide encoding a chimeric antigen receptor (CAR) protein having a target-binding domain that binds to epidermal growth factor receptor (EGFR), a transmembrane domain, and an intracellular signaling domain, wherein the target-binding domain is a ligand for EGFR.
[2] The polynucleotide according to [1], wherein the target-binding domain is epidermal growth factor (EGF) or transforming growth factor-α (TGF-α).
[3] The polynucleotide according to [1] or [2], wherein the target-binding domain is: a polypeptide consisting of an amino acid sequence set forth in SEQ ID NO: 2 or 4; or a polypeptide that comprises an amino acid sequence having a sequence identity of at least 90% with an amino acid sequence set forth in SEQ ID NO: 2 or 4 and has an ability to bind to EGFR.
[4] A vector comprising the polynucleotide according to any of [1] to [3].
[5] A genetically modified cell having the polynucleotide according to any of [1] to [3] or the vector according to [4] introduced thereinto.
[6] A method for producing a CAR protein-expressing cell, comprising introducing the polynucleotide according to any of [1] to [3] or the vector according to [4] into a cell.
[7] A therapeutic agent for a disease involving an EGFR-expressing cell, comprising the cell according to [5].
[8] A pharmaceutical composition comprising the therapeutic agent according to [7] and a pharmaceutically acceptable carrier.
[9] The therapeutic agent according to [7] or the pharmaceutical composition according to [8], wherein the disease involving an EGFR-expressing cell is selected from lung cancer, esophageal cancer, gastric cancer, pancreatic cancer, biliary tract cancer, colon cancer, rectal cancer, head and neck cancer, glioblastoma, brain cancer, breast cancer, ovarian cancer, squamous cell cancer, and adenocarcinoma.
[10] A kit for producing a CAR protein-expressing cell targeting an EGFR-expressing cell, wherein the kit comprises the vector according to [4].

The present specification encompasses the contents disclosed in Japanese Patent Application No. 2022-088480 on which the priority of the present application is based.

### Advantageous Effects of Invention

The present invention provides genetically modified cells that bind to target cells expressing EGFR and exert antitumor effects.

### Brief Description of Drawings

[Figure 1] Figure 1 shows a vector map of an EGF-type CAR expression plasmid.
[Figure 2] Figure 2 shows a vector map of a TGF-α-type CAR expression plasmid.
[Figure 3] Figure 3 shows (A) CAR expression rates and (B) T cell numbers for cell populations having an EGF-type CAR expression plasmid or a TGF-α-type CAR expression plasmid introduced thereinto.
[Figure 4] Figure 4 shows results of flow cytometry when control T cells, EGF-type CAR-T cells, or TGF-α-type CAR-T cells were cocultured with the non-small cell lung cancer cell A549 strain for 5 days.
[Figure 5] Figure 5 shows results of flow cytometry when control T cells, EGF-type CAR-T cells, or TGF-α-type CAR-T cells were cocultured with the non-small cell lung cancer cell H1975 strain for 5 days.
[Figure 6] Figure 6 shows results of flow cytometry when control T cells, EGF-type CAR-T cells, or TGF-α-type CAR-T cells were cocultured with the non-small cell lung cancer cell HCC827 strain for 5 days.
[Figure 7] Figure 7 shows tumor cell numbers when control T cells, EGF-type CAR-T cells, or TGF-α-type CAR-T cells were cocultured with the non-small cell lung cancer cell A549 strain for 5 days.
[Figure 8] Figure 8 shows tumor cell numbers when control T cells, EGF-type CAR-T cells, or TGF-α-type CAR-T cells were cocultured with the non-small cell lung cancer cell H1975 strain for 5 days.
[Figure 9] Figure 9 shows tumor cell numbers when control T cells, EGF-type CAR-T cells, or TGF-α-type CAR-T cells were cocultured with the non-small cell lung cancer cell HCC827 strain for 5 days.
[Figure 10] Figure 10 shows (A) IFN-y concentrations and (B) IL-2 concentrations in culture solutions when control T cells, EGF-type CAR-T cells, or TGF-α-type CAR-T cells were cocultured with non-small cell lung cancer cells (A549 strain, H1975 strain, or HCC827 strain) for 24 hours.
[Figure 11] Figure 11 shows a vector map of a CD19 CAR expression plasmid.
[Figure 12] Figure 12 shows residual tumor volumes in immunodeficient mice subcutaneously inoculated with the non-small cell lung cancer cell HCC827 strain and then subjected to intravenous injection of CD19 CAR-T cells, EGF-type CAR-T cells, or TGF-α-type CAR-T cells. Figure 12A is a series of images of mice obtained by luminescence imaging. Figure 12B shows quantified bioluminescence.
[Figure 13] Figure 13 shows proportions of human T cells in the bloods of immunodeficient mice subcutaneously inoculated with the non-small cell lung cancer cell HCC827 strain and then subjected to intravenous injection of CD19 CAR-T cells, EGF-type CAR-T cells, or TGF-α-type CAR-T cells. Figure 13A shows proportions of T cells on day 28 after T cell injection. Figure 13B shows proportions of T cells on day 63 after T cell injection.

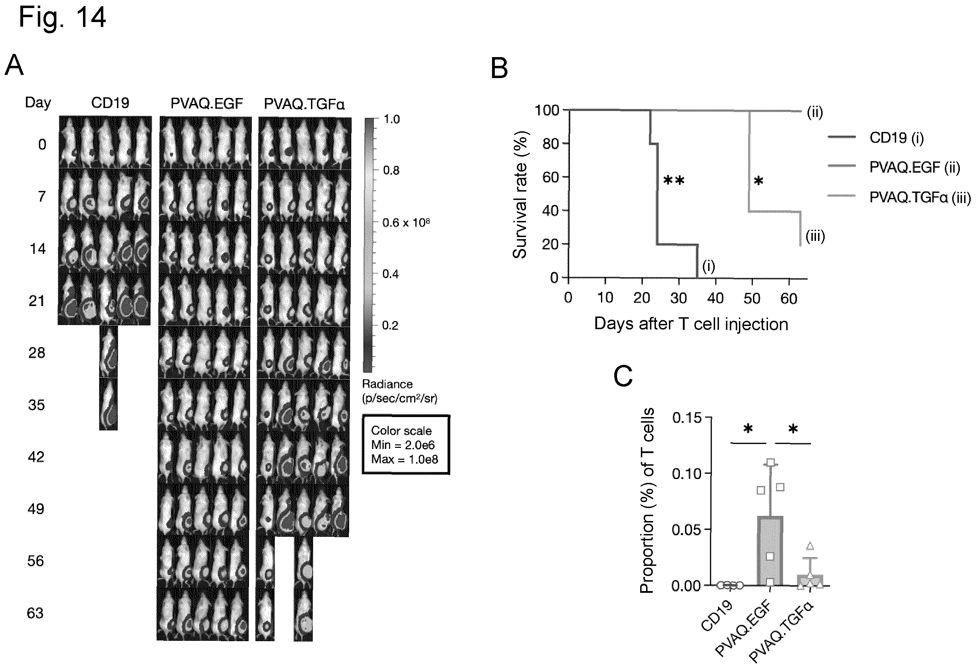

[Figure 14] Figure 14 shows (A) images indicative of residual tumor volume obtained by luminescence imaging, (B) survival rates, and (C) proportions of human T cells in blood for immunodeficient mice subcutaneously inoculated with the non-small cell lung cancer cell H1975 strain and then subjected to intravenous injection of CD19 CAR-T cells, PVAQ.EGF-type CAR-T cells, or PVAQ.TGF-α-type CAR-T cells. In Figure 14B, * (asterisk) and ** indicate the presence of significant difference in log-rank test (indicating p < 0.05 and p < 0.01, respectively). In Figure 14C, * indicates the presence of significant difference in one-way analysis of variance and Tukey's post-hoc test (p < 0.05).
[Figure 15] Figure 15 shows tumor cell numbers when control T cells or EGF-type CAR-T cells were cocultured with (A) the non-small cell lung cancer cell line H460 or (B) the non-small cell lung cancer cell line RERF-LC-Sq1 for 5 days.

### Description of Embodiments

Hereinafter, the present invention will be described in detail.

The present invention provides a polynucleotide encoding a chimeric antigen receptor (CAR) protein having a target-binding domain that binds to epidermal growth factor receptor (EGFR), a transmembrane domain, and an intracellular signaling domain.

As used herein, the "chimeric antigen receptor (CAR)" refers to a modified receptor, which can impart its target specificity to cells such as T cells (e.g., T cells such as naive T cells, stem cell memory T cells, central memory T cells, effector memory T cells or a combination thereof). CAR is also known as an artificial T cell receptor, a chimeric T cell receptor or a chimeric immunoreceptor.

As used herein, the term "encoding" means that a predetermined nucleotide sequence has a code for information on the amino acid sequence of a predetermined protein or (poly)peptide, as usually used in the art. Both a sense strand and an antisense strand are used herein in the context of "encoding".

As used herein, the "polynucleotide" includes, but is not limited to, natural or synthetic DNA and RNA, for example, genomic DNA, cDNA (complementary DNA), mRNA (messenger RNA), rRNA (ribosome RNA), shRNA (small hairpin RNA), snRNA (small nuclear RNA), snoRNA (small nucleolar RNA), miRNA (microRNA), and/or tRNA.

In the present invention, the CAR protein has a target-binding domain that binds to epidermal growth factor receptor (EGFR), a transmembrane domain, and an intracellular signaling domain.

As used herein, the "domain" refers to a region that is within a polypeptide and is folded into a specific structure, independently of other regions.

As used herein, the "epidermal growth factor receptor (EGFR)" is a transmembrane tyrosine kinase receptor, and composed of an extracellular region, a transmembrane region, and a cytoplasmic region including a kinase domain. When a ligand (such as EGF and TGF-α) binds to EGFR, the dimerization of the receptor and the phosphorylation of tyrosine residues occur to activate Ras/Raf/MEK/ERK, PI3K/Akt/mTOR, JAK/STAT, and PKC signaling pathways, causing cell growth, avoidance of apoptosis, angiogenesis, transfer, and so on.

EGFR is known to be expressed in a wide range of tumors. Examples of EGFR-expressing tumors include, but are not limited to, lung cancer (e.g., squamous cell cancer, adenocarcinoma, and large cell cancer which are non-small cell lung cancer), esophageal cancer, gastric cancer, pancreatic cancer, biliary tract cancer, colon cancer, rectal cancer, head and neck cancer, glioblastoma, brain cancer, breast cancer, ovarian cancer, squamous cell cancer, and adenocarcinoma. The amino acid sequence of EGFR protein is registered, for example, as Uniprot Accession No. P00533.

In the present invention, the target-binding domain that binds to EGFR exhibits the ability to bind to EGFR (preferably, its extracellular ligand-binding domain), and enables an immune response to target cells expressing EGFR on the cell surface. In the present invention, the target-binding domain that binds to EGFR is, for example, a ligand for EGFR. Examples of ligands for EGFR include epidermal growth factor (EGF), transforming growth factor-α (TGF-α), amphiregulin, heparin-binding EGF-like growth factor (HB-EGF), betacellulin (BTC), epiregulin, and epigen. Among these ligands for EGFR, EGF and TGF-α have high affinity with EGFR. In addition, EGF and TGF-α specifically bind to EGFR, thus inferred to have less off-target toxicity. Accordingly, EGF and TGF-α are particularly preferred as the ligand for EGFR for use in the present invention. The ligand for EGFR such as EGF or TGF-α can be derived from any mammals including humans, domestic animals (horses, cows, sheep, goats, pigs, etc.), pet animals (dogs, cats, rabbits, etc.), and experimental animals (mice, rats, monkeys, etc.), and is preferably derived from a human. Examples of the amino acid sequence of naturally occurring human EGF include that set forth in SEQ ID NO: 2, and examples of the nucleotide sequence encoding it include that set forth in SEQ ID NO: 1. Examples of the amino acid sequence of naturally occurring human TGF-α include that set forth in SEQ ID NO: 4, and examples of the nucleotide sequence encoding it include that set forth in SEQ ID NO: 3.

In the present invention, the naturally occurring EGF or TGF-α, or a variant thereof can be used as the target-binding domain. Accordingly, the target-binding domain can be a polypeptide consisting of an amino acid sequence set forth in SEQ ID NO: 2 or 4, or a polypeptide that comprises or consists of an amino acid sequence having a sequence identity of at least 90%, at least 91%, at least 92%, at least 93%, at least 94%, at least 95%, at least 96%, at least 97%, at least 98%, at least 99%, or 100% with an amino acid sequence set forth in SEQ ID NO: 2 or 4 and retains an ability to bind to EGFR.

By the phrase "the ability of the target-binding domain to bind to EGFR", it is meant that the KD value is, for example, 100 nM or less, preferably 10 nM or less, and more preferably 5 nM or less. The ability to bind may be relatively weak as compared with antigen-antibody binding ability.

In the present invention, the CAR protein can optionally comprise an "extracellular spacer domain" between the target-binding domain, which is extracellularly present, and the transmembrane domain. The extracellular spacer domain is desirably a sequence that promotes the binding of a CAR to the antigen and facilitates signal transduction into a cell. For example, an Fc fragment of an antibody or a fragment or a derivative thereof, a hinge region of an antibody or a fragment or a derivative thereof, a CH2 region of an antibody, a CH3 region of an antibody, an artificial spacer sequence, or a combination thereof can be used.

In one aspect of the present invention, (i) hinge, CH2, and CH3 regions of IgG4, (ii) a hinge region of IgG4, (iii) a hinge and CH2 of IgG4, (iv) a hinge region of CD8a, (v) hinge, CH2 and CH3 regions of IgG1, (vi) a hinge region of IgG1, or (vii) a hinge and CH2 of IgG1, or a combination thereof can be used as the extracellular spacer domain. For example, a region having the following amino acid sequence (SEQ ID NO: 5) can be suitably used as the hinge region of IgG1, though the hinge region is not limited thereto.
UniProt No.: P01857 (99-110)

| | | |
|---|---|---|
| EPKSCDKTHTCP | PCDPA | EPKSPDKTHTCP |
| Hinge | Spacer | Hinge |

A region having the amino acid sequence set forth in SEQ ID NO: 6 and a region having the amino acid sequence set forth in SEQ ID NO: 7 can be suitably used as the CH2 region and the CH3 region, respectively, of IgG1. To prevent CAR-T cells from being taken up by the lung (captured by alveolar macrophages) through binding of the CH2 region of IgG1 to the Fcy receptor, a mutation that reduces binding to the Fcy receptor may be introduced into the CH2 region of IgG1. Examples of the mutation include substitution of ELLG at positions 6 to 9 of SEQ ID NO: 6 with PVA and substitution of N at position 70 of SEQ ID NO: 6 with Q (Watanabe, N. et al., OncoImmunology, 2016, Vol. 5, No. 12, e1253656).

In a preferred aspect, hinge, CH2, and CH3 regions of human IgG1 or a portion thereof can be used as the extracellular spacer domain.

In a preferred aspect, (i) a human IgG1 hinge region (SEQ ID NO: 5) alone, (ii) a combination of a human IgG1 hinge region (SEQ ID NO: 5), CH2 region (SEQ ID NO: 6) and CH3 region (SEQ ID NO: 7), (iii) a combination of a human IgG1 hinge region (SEQ ID NO: 5) and CH3 region (SEQ ID NO: 7), (iv) a CH3 region (SEQ ID NO: 7) alone, or (v) a combination of a CH2 region (SEQ ID NO: 6) and CH3 region (SEQ ID NO: 7) can be used as the extracellular spacer domain. For example, the extracellular spacer domain may contain: a CH2 region with or without the mutation that reduces interaction with the Fcy receptor; and a CH3 region. Examples of the amino acid sequence of such an extracellular spacer domain include SEQ ID NOs: 14 and 15.

In one aspect of the present invention, a spacer sequence represented by the formula (G4S)n can be used as the artificial spacer sequence for use in the extracellular spacer domain. In the formula, n is 1 to 10, preferably n = 3. A spacer having such a spacer sequence is also called peptide linker. A peptide linker suitably used in the art can be appropriately used in the present invention. In this case, the configuration and chain length of the peptide linker can be properly selected without impairing the function of the resulting CAR protein.

The extracellular spacer domain is not particularly limited, but can be appropriately selected from those listed above or further modified on the basis of common general technical knowledge in the art, and used for the present invention.

Nucleotide sequences encoding the respective amino acid sequences of domains can be ligated, inserted into a vector, and expressed in a host cell such that the extracellular spacer domain can reside between the target-binding domain and the transmembrane domain. Alternatively, the extracellular spacer domain may be modified using a polynucleotide encoding a plasmid CAR protein produced in advance as a template.

The modification of the extracellular spacer domain is useful when taking into consideration, for example, improvement in the CAR gene expression rate in a host cell of a CAR-T cell having a polynucleotide encoding CAR introduced thereinto, signal transduction, aging of cells, distribution in tumor, antigen recognition or influence on *in vivo* activity.

In the present invention, the CAR protein comprises: an extracellular domain comprising a target-binding domain and optionally an extracellular spacer domain; a transmembrane domain; and intracellular domain comprising an intracellular signaling domain and optionally a costimulatory domain. As well known in the art, the "transmembrane domain" is a domain having an affinity to a lipid bilayer constituting a cell membrane, whereas the extracellular domain and intracellular domain are both hydrophilic domains.

The transmembrane domain is not particularly limited as long as the CAR protein can reside on the cell membrane without impairing the functions of the target-binding domain and the intracellular signaling domain. A polypeptide derived from the same protein as that of a costimulatory domain mentioned later may function as the transmembrane domain. A transmembrane domain such as CD28, CD3ε, CD8α, CD3, CD4 or 4-1BB can be used as the transmembrane domain. For example, human CD28 (Uniprot No.: P10747 (153-179)) can be used as the transmembrane domain. Specifically, a domain having an amino acid sequence encoded by the nucleotide sequence of NCBI Accession No.: NM_006139.3 (679-759) can be suitably used as the transmembrane domain.

The CAR protein can optionally comprise a "costimulatory domain". The costimulatory domain specifically binds to a costimulatory ligand, thereby mediating cellular costimulation responses such as growth of CAR-T cells, cytokine production, functional differentiation and cell death of target cells, though the cellular co-stimulation responses are not limited thereto. For example, CD27, CD28, 4-1BB (CD137), CD134 (OX40), Dap10, CD2, CD5, CD30, CD40, PD-1, ICAM-1, LFA-1 (CD11a/CD18), TNFR-1, TNFR-II, Fas, or Lck can be used as the costimulatory domain. For example, human CD28 (Uniprot No.: P10747 (180-220)) or 4-1BB (GenBank: U03397.1) can be used as the costimulatory domain. Specifically, a domain having an amino acid sequence encoded by the nucleotide sequence of NCBI Accession No.: NM_006139.3 (760-882) can be suitably used as the costimulatory domain.

In the case of using a transmembrane domain and a costimulatory domain both human CD28-derived, for example, those having the amino acid sequence set forth in SEQ ID NO: 8 can be used.

The CAR protein comprises an "intracellular signaling domain". The intracellular signaling domain transduces signals required for exerting the effector function of immunocytes. For example, a human CD3ζ chain, FcyRIII, FcεRI, a cytoplasmic end of an Fc receptor, a cytoplasmic receptor having an immunoreceptor tyrosine activation motif (ITAM) or a combination thereof can be used as the intracellular signaling domain. For example, a human CD3ζ chain (e.g., nucleotides 299-637 of NCBI Accession No. NM_000734.3) can be used as the intracellular signaling domain. Specifically, a domain having an amino acid sequence set forth in SEQ ID NO: 9 can be suitably used as the intracellular signaling domain.

For the purpose of promoting secretion of the CAR protein, a leader sequence to introduce the transfer of the protein at or after translation is appropriately included at the N terminus of the protein. Examples of useful leader sequences that can be suitably used in the present invention can include, but are not limited to, human immunoglobulin (Ig) heavy chain signal peptide, CD8α signal peptide, or human GM-CSF receptorα signal peptide. For the Ig heavy chain signal peptide, for example, one derived from IgG1, IgG2, IgG3, IgA1, or IgM can be suitably used. For example, a leader sequence having the amino acid sequence set forth in SEQ ID NO: 13 can be used.

The polynucleotide of interest can be easily produced according to a routine method. Nucleotide sequences encoding the respective amino acid sequences of domains can be obtained from NCBI RefSeq ID or GenBank Accession numbers indicating the amino acid sequences. The polynucleotide of the present invention can be produced using standard molecular biological and/or chemical procedures. For example, nucleic acids can be synthesized on the basis of these nucleotide sequences. Also, DNA fragments obtained through the polymerase chain reaction (PCR) from a cDNA library can be combined to produce the polynucleotide of the present invention.

Thus, the polynucleotide encoding the CAR protein can be produced by ligating respective polynucleotides encoding the domains described above. A genetically modified cell can be produced by introducing this polynucleotide to a proper cell. Alternatively, the CAR protein may be produced by using a polynucleotide encoding a known CAR protein having the same constituents except for the target-binding domain, as a template, and recombining the target-binding domain according to a routine method.

Depending on the purpose, one or more domains, for example, the extracellular spacer domain, can be modified by use of inverse-PCR (iPCR), etc. using a polynucleotide encoding a known CAR protein as a template. The technique for modifying the extracellular spacer domain is described in, for example, Oncoimmunology, 2016, Vol. 5, No. 12, e1253656.

The method for introducing the polynucleotide for the production of the genetically modified cell is not particularly limited as long as the method is usually used. In the case of introducing the polynucleotide using a vector, examples of the vector that may be used include, but are not particularly limited to, lentivirus vectors, retrovirus vectors, foamy virus vectors and adeno-associated virus vectors. Introduction of the polynucleotide can be carried out in a non-viral manner with a transposon method. Plasmid transposon can be used for the transposon method, and a sleeping beauty transposon system (e.g., described in Huang X, Guo H, et al. Mol Ther. 2008; 16: 580-9; Singh H, Manuri PR, et al. Cancer Res. 2008; 68: 2961-71; Deniger DC, Yu J, et al. PLoS One. 2015; 10: e0128151; Singh H, Moyes JS, et al. Cancer Gene Ther. 2015; 22: 95-100; Hou X, Du Y, et al. Cancer Biol Ther. 2015; 16: 8-16; Singh H, Huls H, et al. Immunol Rev. 2014; 257: 181-90; and Maiti SN, Huls H, et al. J Immunother. 2013; 36: 112-23) or a piggyBac transposon system (described in Nakazawa Y, Huye LE, et al. J Immunother. 2009; 32: 826-36; Galvan DL, Nakazawa Y, et al. J Immunother. 2009; 32: 837-44; Nakazawa Y, Huye LE, et al. Mol Ther. 2011; 19: 2133-43; Huye LE, Nakazawa Y, et al. Mol Ther. 2011; 19: 2239-48; Saha S, Nakazawa Y, et al. J Vis Exp. 2012; (69): e4235; Nakazawa Y, Saha S, et al. J Immunother. 2013; 36: 3-10; Saito S, Nakazawa Y, et al. Cytotherapy. 2014; 16: 1257-69; and Nakazawa et al. Journal of Hematology & Oncology (2016) 9:27) can be suitably used.

In the case of using the piggyBac transposon system, a plasmid retaining a gene encoding piggyBac transposase (herein, referred to as a piggyBac plasmid) and a plasmid having a structure in which a polynucleotide encoding the CAR protein is sandwiched between piggyBac reverse repetitive sequences are typically introduced (transfection). For the transfection, various techniques including electroporation, nucleofection, lipofection, and a calcium phosphate method can be used. Each of the plasmids can contain a poly A-added leader sequence, a reporter gene, a selection marker gene, an enhancer sequence, and so on.

Examples of applicable apparatuses for electroporation include, but are not limited to, a 4D-Nucleofector (Lonza Japan Ltd.), NEPA21 (Nepa Gene Co., Ltd.), and a Maxcyte GT (Maxcyte, Inc.), and each can be operated in accordance with the attached instruction manual. The above method enables, for example, gene introduction into 1 × 10⁶ to 2 × 10⁷ cells.

T cells or a cell population containing T cells derived from a mammal, for example, cells derived from a human, or a non-human mammal, such as a monkey, a mouse, a rat, a pig, a cow or a dog can be used as the cells into which the polynucleotide described above is to be introduced, and use of cells releasing a cytotoxic protein (perforin, granzyme, etc.) is preferred. Specifically, for example, a cell population containing T cells, progenitor cells of T cells (hematopoietic stem cells, lymphoid progenitor cells, etc.), or NK-T cells can be used. Further, cells capable of differentiating into these cells include various stem cells such as ES cells and iPS cells. The T cells include CD8-positive T cells, CD4-positive T cells, regulatory T cells, cytotoxic T cells or tumor-infiltrating lymphocytes. The cell population containing T cells and precursor cells of T cells includes PBMC. The cells described above may be collected from a living organism, obtained by the expansion culture of the cells, or established as a cell line. In the case of transplanting the produced cells expressing CAR or cells differentiated from the cells into a living organism, it is desirable to introduce the nucleic acid into cells collected from the living organism itself or a living organism of the same species thereas.

T cells expected to have a sustained antitumor effect, for example, stem cell memory T cells, can be used as T cells for use in adoptive immunotherapy to which the polynucleotide is to be introduced for the genetically modified cell of the present invention. The stem cell memory T cells can be analyzed according to a routine method and easily confirmed as described in, for example, Yang Xu, et al. Blood. 2014; 123:3750-3759.

In one embodiment, for example, CD45R0⁻, CD62L⁺, CD45RA⁺, and CCR7⁺ T cells can be used as the stem cell memory T cells.

The present invention also provides a vector comprising the polynucleotide of the present invention.

The present invention also provides a genetically modified cell having the polynucleotide of the present invention or the vector of the present invention introduced thereinto. The genetically modified cell of the present invention can express the CAR protein that binds to EGFR-expressing cells, on the cell membrane.

As demonstrated in Examples shown later, the genetically modified cell (CAR protein-expressing cell) of the present invention can cause immune response not only to cells expressing wild-type EGFR but also to cells expressing EGFR having L858R and T790M mutations (known to be unaffected by first-generation EGFR tyrosine kinases) and cells expressing EGFR having deletion of E746 to A750 in exon 19.

In Examples shown later, CAR-T cells expressing CAR comprising a ligand for EGFR as the target-binding domain were produced and administered to tumor-bearing mice, and as a result antitumor effects of CAR-T cells were observed, but no discernable toxicity was observed. The present inventors have confirmed that even in cynomolgus monkeys depleted of lymphocytes, CAR-T cells expressing CAR comprising a ligand for EGFR as the target-binding domain exhibited no discernable toxicity. Therefore, the genetically modified cell of the present invention can exhibit an antitumor effect without any adverse effect.

Furthermore, the present invention provides a method for producing a CAR protein-expressing cell, comprising introducing the polynucleotide of the present invention or the vector of the present invention into a cell.

CAR protein-expressing cells can be cultured and proliferated by any means without particular limitation. For example, a polynucleotide encoding a CAR protein can be introduced into a cell in the manner described above, and non-specific or CAR-specific stimuli can then be applied to the cell, for the purpose of activation or the like of the CAR protein-expressing cell. A method of cell stimulation is not limited. As a non-specific cell stimulation, for example, stimuli can be applied using anti-CD3 antibody and/or anti-CD28 antibody. As a CAR-specific stimulation, for example, stimuli can be applied using artificial antigen presenting cells (aAPCs) obtained by expressing CAR-binding antigen molecules or costimulatory factors in tumor cell lines such as K562. While culture conditions are not particularly limited, for example, culture is suitably carried out at 37°C and 5% CO₂ for 1 to 15 days or 1 to 10 days.

In the method of the present invention described above, a method of introducing a polynucleotide into a cell is not limited, and the transposon method is suitably employed. As the transposon method, the transposon system described above or other systems suitable in the present invention may be employed. The method of the present invention can be suitably performed by the piggyBac method, although the method is not limited thereto.

In another aspect of non-specific stimulation, after a cell population comprising T cells is stimulated by one or more types of viral peptide antigens, cells in which the viral growth ability is inactivated by a conventional technique are used as feeder cells to promote activation of cells into which CAR has been introduced. As viral peptide antigens, for example, an AdV antigen peptide mixture, a CMV antigen peptide mixture, an EBV antigen peptide mixture, or a combination thereof can be used.

In another aspect of specific stimulation, CAR-expressing cells can be cocultured with immature dendritic cells produced by culturing PBMCs derived from the individual from which the CAR-expressing cells are derived in a medium containing human IL-4 and GM-CSF, as described in WO 2020/085480. In another aspect of specific stimulation, a vector for overexpressing EGFR or a fragment containing its extracellular domain, CD80, and 4-1-BBL is introduced into PBMCs derived from the individual from which CAR-expressing cells are derived, and the resulting cells and the CAR-expressing cells can be cocultured, as described in Nakamura, K. et al., Mol. Ther. Methods Clin. Dev., 2021, 21, 315-324. The amino acid sequences of the extracellular domain of EGFR, CD80, and 4-1-BBL can be, for example, those set forth in SEQ ID NOs: 18, 19, and 20, respectively.

In order to enhance cell viability and proliferation rate, culture can be carried out in the presence of one or more types of cytokines. For example, culture can be preferably carried out in the presence of cytokines, such as IL-7 and IL-15.

The present invention further provides a kit for producing a CAR protein-expressing cell targeting an EGFR-expressing cell, wherein the kit comprises the vector of the present invention. The kit of the present invention can appropriately contain, for example, a reagent, a buffer, a reaction vessel, and instructions that are necessary for producing a CAR protein-expressing cell. The kit of the present invention can be suitably used for producing the genetically modified cell of the present invention.

The cell of the present invention causes a receptor-specific immune response to target cells expressing EGFR on a surface and is thereby activated through signal transduction into the cell. The activation of the cell expressing CAR differs depending on the type of the host cell or the intracellular domain of CAR and can be confirmed using, for example, cytokine release, improvement in the cell growth rate, or change in the cell surface molecule as an index. The release of a cytotoxic protein (perforin, granzyme, etc.) brings about the destruction of cells expressing the receptor.

The genetically modified cell of the present invention can be used as a therapeutic agent for a disease involving an EGFR-expressing cell (e.g., cancer expressing EGFR). Thus, the present invention provides a therapeutic agent for a disease involving an EGFR-expressing cell, comprising the genetically modified cell of the present invention. The disease that is expected to be treated with the therapeutic agent of the present invention is not limited as long as the disease exhibits sensitivity to the cell. Examples thereof include diseases involving a cell expressing EGFR on the cell membrane (cancer, etc.) such as lung cancer (e.g., squamous cell cancer, adenocarcinoma, and large cell cancer which are non-small cell lung cancer), esophageal cancer, gastric cancer, pancreatic cancer, biliary tract cancer, colon cancer, rectal cancer, head and neck cancer, glioblastoma, brain cancer, breast cancer, ovarian cancer, squamous cell cancer, and adenocarcinoma.

In one aspect, the therapeutic agent of the present invention is an anticancer agent for EGFR-expressing tumor cells such as the above tumors. The therapeutic agent or the anticancer agent of the present invention may be used alone or can be used in combination with an agent and/or treatment differing in mechanism therefrom.

Thus, the therapeutic agent of the present invention can be used, either alone or in combination with an additional active ingredient, in the form of a pharmaceutical composition. The therapeutic agent or the pharmaceutical composition of the present invention can be topically or systemically administered and is not limited by its dosage form. For example, intravenous administration is preferred. The pharmaceutical composition can comprise, in addition to the therapeutic agent of the present invention and the additional active ingredient, a carrier, an excipient, a buffer and a stabilizer, and the like which are usually used in the art, according to the dosage form. In one aspect of the present invention, for example, the pharmaceutical composition can comprise the therapeutic agent of the present invention and a pharmaceutically acceptable carrier.

The dose of the therapeutic agent of the present invention varies depending on the body weight and age of the patient, the severity of the disease, etc. and is not particularly limited. For example, the therapeutic agent of the present invention can be administered in the range of 10⁴ to 10¹⁰ CAR-positive cells/kg body weight, or 10⁶ to 10⁸ CAR-positive cells/kg body weight, once to several times per day, every 2 days, every 3 days, weekly, every 2 weeks, monthly, every 2 months or every 3 months.

Examples of subjects to which the therapeutic agent or pharmaceutical composition of the present invention is administered include any mammals including humans, domestic animals (horses, cows, sheep, goats, pigs, etc.), pet animals (dogs, cats, rabbits, etc.), and experimental animals (mice, rats, monkeys, etc.), and humans are preferred. Subjects to which the therapeutic agent or pharmaceutical composition of the present invention is administered may have tumor expressing wild-type EGFR or have tumor expressing mutated EGFR. Examples of the mutated EGFR include EGFR having a mutation selected from the group consisting of L858R mutation, T790M mutation, and deletion of E746 to A750 in exon 19 to wild-type EGFR.

The CAR expression rate of cells (CAR-positive CD3-positive cell rate) contained in the therapeutic agent or pharmaceutical composition of the present invention can be 30% or more, 40% or more, or 50% or more. The proportion of CD62L⁺CD45RA⁺ cells in CAR-positive cells contained in the therapeutic agent or pharmaceutical composition of the present invention can be, for example, 90% or more, 95% or more, or 99% or more. The proportion of CD4⁺ cells in CAR-positive cells contained in the therapeutic agent or pharmaceutical composition of the present invention can be, for example, 10 to 90%, 30 to 80%, or 50 to 70%. The proportion of CD8⁺ cells in CAR-positive cells contained in the therapeutic agent or pharmaceutical composition of the present invention can be, for example, 10 to 90%, 15 to 70%, or 20 to 50%.

Furthermore, the present invention provides a method for treating a disease involving an EGFR-expressing cell, comprising administering a therapeutically effective amount of the therapeutic agent or pharmaceutical composition of the present invention to a patient. The therapeutically effective amount and administration regimen can be appropriately determined in view of various factors as described above.

### Examples

Hereinafter, the present invention will be described further specifically with Examples. However, the technical scope of the present invention is not limited by Examples.

### [Example 1 Production of EGF- and TGF-α-type CAR expression plasmids]

Plasmids for expressing CAR comprising EGF or TGF-α as a target-binding domain (EGF- or TGF-α-type CAR expression plasmid) were produced according to the method described in WO 2020/085480.

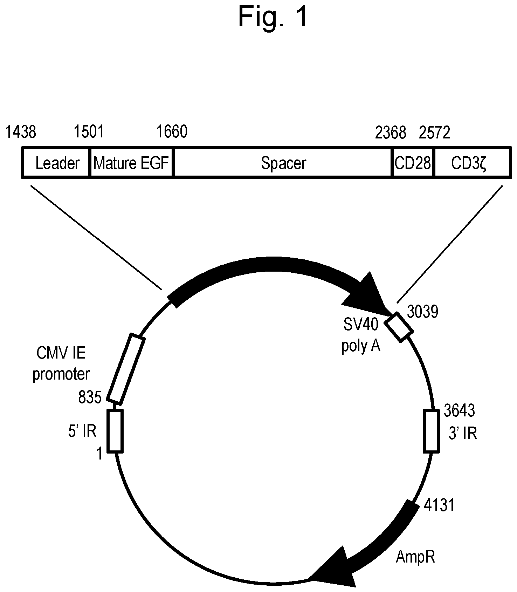

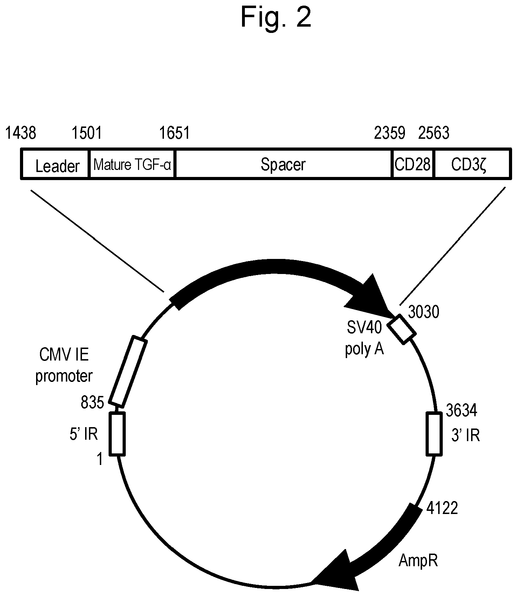

The vector maps of the plasmids produced are shown in Figures 1 and 2.

The EGF-type CAR expression plasmid (SEQ ID NO: 10) contains a CAR construct consisting of a nucleotide sequence encoding a leader sequence (positions 1438 to 1500 of SEQ ID NO: 10), human mature EGF (positions 1501 to 1659 of SEQ ID NO: 10), a spacer (positions 1660 to 2367 of SEQ ID NO: 10), CD28 (position 2368 to position 2571 of SEQ ID NO: 10), and CD3ζ (position 2572 to position 2910 of SEQ ID NO: 10) (Figure 1).

The TGF-α-type CAR expression plasmid (SEQ ID NO: 11) is the same as the EGF-type CAR expression plasmid except that it contains a nucleotide sequence encoding human mature TGF-α instead of human mature EGF (Figure 2).

The nucleotide sequences of human mature EGF and TGF-α used for the CAR expression plasmids are set forth in SEQ ID NOs: 1 and 3, respectively. The amino acid sequences encoded by them are shown in SEQ ID NOs: 2 and 4, respectively. The amino acid sequences of the leader sequence, spacer, CD28, and CD3ζ used for the CAR expression plasmids are set forth in SEQ ID NOs: 13, 14, 8, and 9, respectively.

### [Example 2 Culture and amplification of EGF- and TGF-α-type CAR-T cells]

Using the CAR expression plasmids produced in Example 1, CAR-T cells with EGF or TGF-α as a target-binding domain (EGF-type CAR-T cells and TGF-α-type CAR-T cells) were produced, and cultured and amplified.

### <2-1 Isolation of PBMCs (Day 0)>

Peripheral blood mononuclear cells (PBMCs) were isolated from the peripheral blood of a healthy volunteer donor using Ficoll-PaquePlus (GE Healthcare, Chicago, IL).

### <2-2 Gene introduction and culture (Days 0 to 3)>

To 2 × 10⁷ cells of the PBMCs isolated in 2-1 above, 7.5 µg of the CAR expression plasmid produced in Example 1 (EGF-type CAR expression plasmid or TGF-α-type CAR expression plasmid), 7.5 µg of a pCMV-piggyBac plasmid (Matthew H Wilson et al., Molecular Therapy, 2007; 15(1): 139-145), and 100 µl of P3 Primary Cell Nucleofector^{™} solution (Lonza, Basel, Switzerland) with addition of Supplement 1 were added, and electroporation was performed with the apparatus 4D-Nucleofector (program FL-115) (Day 0).

The electroporated PBMCs were suspended in ALyS^{™} 705 culture solution (Cell Science and Technology Institute Inc., Japan) containing 10 ng/mL human IL-7 (Miltenyi Biotec, BergischGladbach, Germany), 5 ng/mL human IL-15 (Miltenyi Biotec, BergischGladbach, Germany), and 5% artificial serum (Artificial serum Animal-free; Cell Science & Technology Institute Inc., Japan), seeded in a 24-well plate, and cultured at 37°C and 5% CO₂ for 3 days (Days 0 to 3).

### <2-3 Production of immature dendritic cells (Days 0 to 3)>

3 × 10⁶ cells of the PBMCs isolated in 2-1 above were suspended in ALyS^{™} 705 culture solution containing 10 ng/mL human IL-4 and 10 ng/mL human GM-CSF, seeded in a G-REX^{R} cell culture plate 6 well (Wilson Wolf, Saint Paul, MN), and cultured at 37°C and 5% CO₂ for 3 days (Days 0 to 3).

### <2-4 Stimulation and culture of transgenic cells with immature dendritic cells (Days 3 to 10)>

A supernatant was removed from the G-REX^{R} cell culture plate containing immature dendritic cells cultured for 3 days in 2-3 above, and ALyS^{™} 705 culture solution containing 10 ng/mL human IL-7, 5 ng/mL human IL-15, and 5% artificial serum (Artificial serum Animal-free) was added to the plate. To this G-REX^{R} cell culture plate, the transgenic cells cultured for 3 days in 2-2 above were added (Day 3), and culture was further continued. During the culture period, half volume of the medium was exchanged every 3 to 4 days, and IL-7 and IL-15 were added to reach final concentrations of 10 ng/mL and 5 ng/mL, respectively.

### <2-5 Production of control T cells (Days 0 to 10)>

1.5 × 10⁶ cells of the PBMCs isolated in 2-1 above were suspended in ALyS^{™} 705 culture solution containing 5 ng/mL IL-15 and 5% artificial serum, and seeded in a 24-well non-treatment plate coated with an anti-CD3 antibody and anti-CD28 antibody (Miltenyi Biotec, Auburn, CA), and culture was started at 37°C and 5% CO₂ (Day 0).

On day 2 after the start of culture (Day 2), the resultant was transferred to a 24-well treatment plate, and culture was further continued. During the culture period, medium exchange was performed every 3 to 4 days.

### <2-6 Evaluation of CAR expression rates (Day 10)>

The CAR expression rates in the cell populations having the EGF-type CAR expression plasmid introduced thereinto and the cell populations having the TGF-α-type CAR expression plasmid introduced thereinto were evaluated by flow cytometry analysis.

The cell populations cultured until Day 10 in 2-4 above were stained with a FITC (fluorescein isothiocyanate)-labeled anti-human IgG antibody and an APC-labeled anti-CD3 antibody. The FITC-labeled anti-human IgG antibody used was Fluorescein (FITC) AffiniPure F(ab')₂ Fragment Goat Anti-Human IgG (H+L) (Jackson ImmunoResearch Inc. West Grove, PA, USA). The APC-labeled anti-CD3 antibody used was CD3 Antibody, anti-human, APC (Miltenyi Biotec, Auburn, CA). The FITC-labeled anti-human IgG antibody binds to the spacer moiety of CAR, and thus, CAR-expressing cells can be detected by the FITC-labeled anti-human IgG antibody. T cells can be detected by the APC-labeled anti-CD3 antibody.

The stained cells were applied to the flow cytometer BD Accuri^{™} C6 Plus (BD Biosciences San Jose, CA) and analyzed with Flowjo (BD Biosciences San Jose, CA), and CAR-positive CD3-positive cell rates were determined as CAR expression rates.

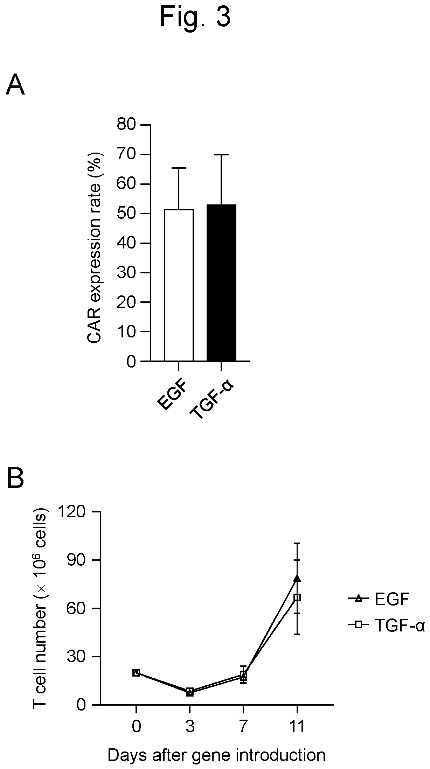

The results are shown in Figure 3A. The CAR expression rates on Day 10 in the cell populations having the EGF-type CAR expression plasmid introduced thereinto and the cell populations having the TGF-α-type CAR expression plasmid introduced thereinto were both 50% or more, and thus excellent CAR expression was found.

### <2-7 Evaluation of T cell numbers (Days 0, 3, 7, and 11)>

The T cell numbers in the cell populations having the EGF-type CAR expression plasmid introduced thereinto and the cell populations having the TGF-α-type CAR expression plasmid introduced thereinto were evaluated by flow cytometry analysis.

The cell populations cultured until Day 0, 3, 7, or 11 in 2-2 or 2-4 above were stained with a FITC-labeled CD3 antibody. Flow cytometry was performed in the same manner as in 2-6 above to determine the T cell numbers in the cell populations.

The results are shown in Figure 3B. Increased T cell numbers were found after day 7 following the gene introduction for both the cell populations having the EGF-type CAR-T expression plasmid introduced thereinto and the cell populations having the TGF-α-type CAR expression plasmid introduced thereinto.

The phenotypes of the CAR-T cells produced were examined by flow cytometry. As a result, the proportions of CD4⁺ cells and CD8⁺ cells in CAR-positive cells were 67.7 ± 7.69% and 29.3 ± 8.13% (mean ± standard deviation; n = 3), respectively, for the EGF-type CAR-T cells, and 53.3 ± 22.3% and 42.7 ± 21.7% (mean ± standard deviation; n = 3), respectively, for the TGF-α-type CAR-T cells. The proportion of CD62L⁺CD45⁺ cells (stem cell memory cells) in CAR-positive cells was more than 99% for both the EGF-type CAR-T cells and the TGF-α-type CAR-T cells.

### [Example 3 In vitro anti-tumor cell activities of EGF- and TGF-α-type CAR-T cells]

To evaluate the anti-tumor cell activities of the EGF-type CAR-T cells and TGF-α-type CAR-T cells produced in Example 2, coculture test was performed with CAR-T cells and tumor cells.

On the day before coculture, 0.25 × 10⁶ cells of the non-small cell lung cancer cell line A549, H1975, or HCC827 (A549-Luc, NCI-H1975-Luc, HCC-827-Luc; the National Institutes of Biomedical Innovation, Health, and Nutrition JCRB Cell Bank) as tumor cells were added to wells of a 24-well plate, and cultured. A549 expresses wild-type EGFR. H1975 expresses EGFR with L858R and T790M mutations. HCC827 expresses activated EGFR with deletion of exon 19.

On the day of coculture, CAR-T cells cultured for 10 days in Example 2 or control T cells (T cells activated with an anti-CD3 antibody and an anti-CD28 antibody) were added to the wells of the 24-well plate to give a T cells:tumor cells (E:T) ratio of 4:1, 2:1, 1:1, 1:2, or 1:4, and coculture was performed at 37°C and 5% CO₂. The culture solution used was RPMI1640 medium with addition of 10% FBS.

After 24 hours from the start of coculture, 1 ml of the culture supernatant was collected from each to measure the cytokine concentrations (IFN-y and IL-2) in the culture supernatant, and the medium in a volume equal to the collected volume was newly added.

After 5 days from the start of coculture, the cells were collected, and stained with a FITC-labeled anti-CD3 antibody (BioLegend, for detection of T cells), an APC-labeled anti-B7-H3 antibody (BioLegend, for detection of tumor cells), and 7-aminoactinomycin D (7-Amino-Actinomycin D; 7-AAD, BD Biosciences, for detection of dead cells).

CountBright absolute Counting Beads (Invitrogen, Carlsbad, CA) were added to the stained cells. The stained cells were then applied to the flow cytometer BD Accuri^{™} C6 Plus (BD Biosciences San Jose, CA), and analyzed on tumor cell rates and tumor cell numbers with Flowjo (BD Biosciences San Jose, CA).

The above experiment was performed for PBMCs derived from each of five donors.

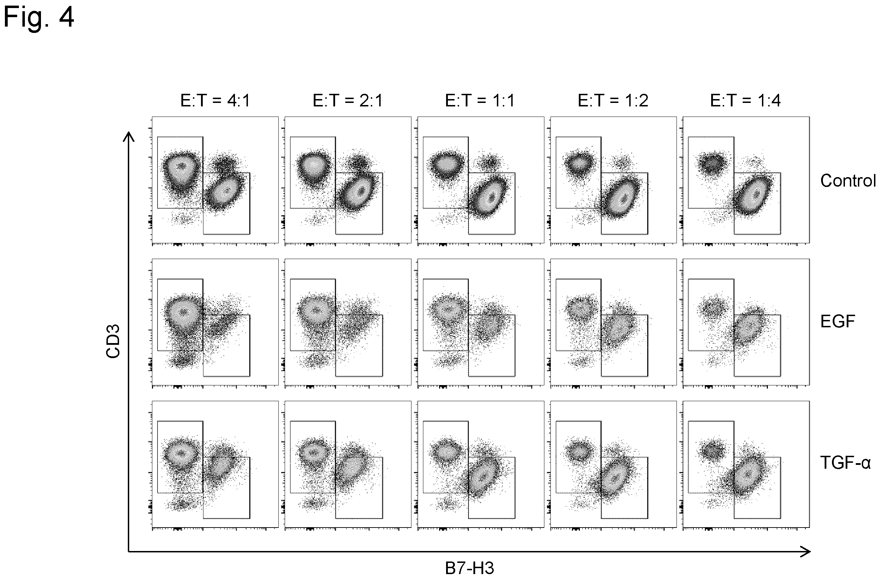

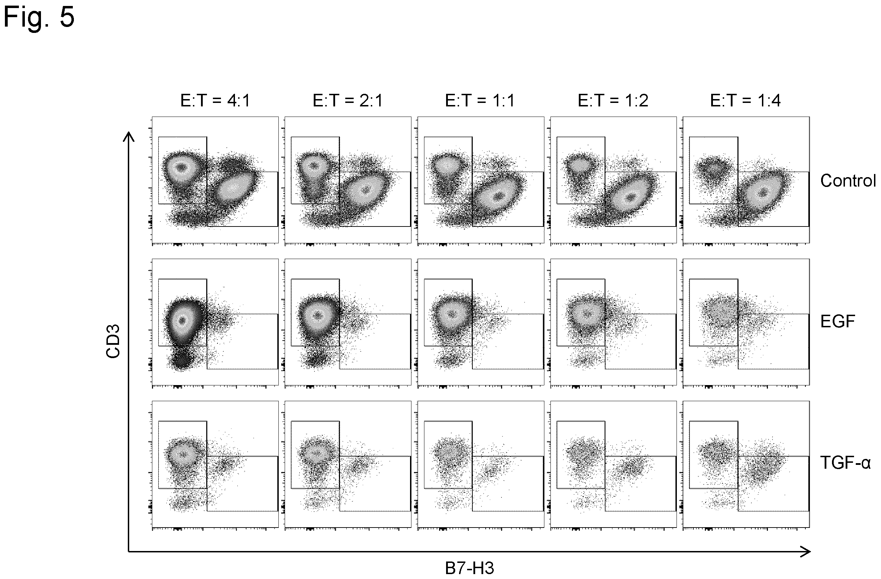

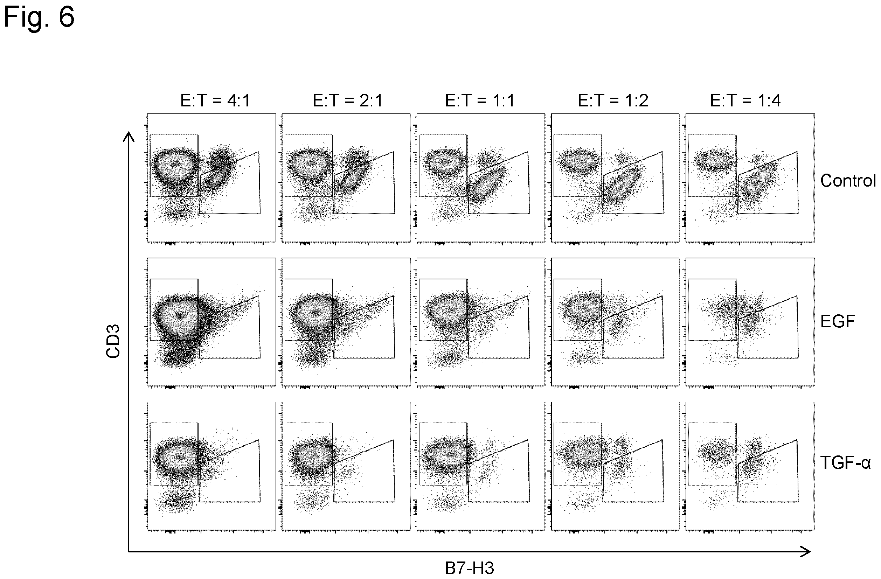

Representative examples of the results of the flow cytometry are shown in Figures 4 to 6. The EGF-type and TGF-α-type CAR-T cells caused more reduction in tumor cell rates than control T cells did at all of the E:T ratios tested.

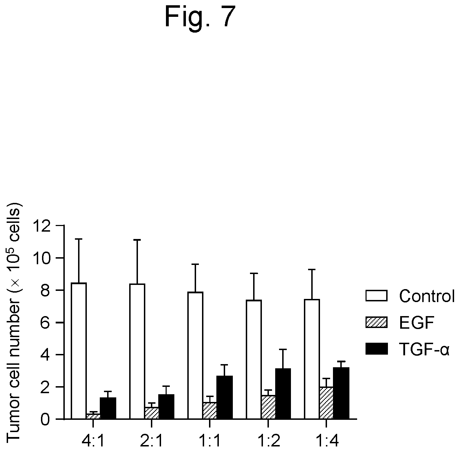

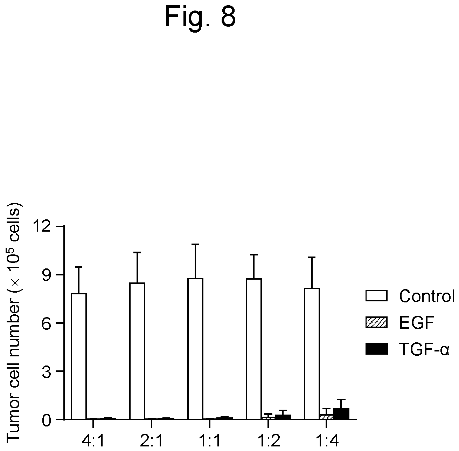

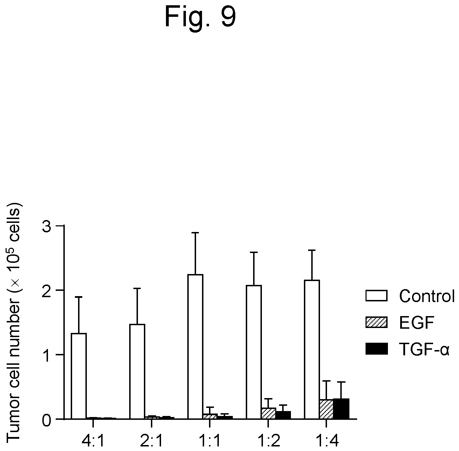

The results of the measurement of tumor cell numbers are shown in Figures 7 to 9. The EGF-type and TGF-α-type CAR-T cells caused more reduction in tumor cell numbers than the control T cells did at all of the E:T ratios tested.

The EGF-type and TGF-α-type CAR-T cells exhibited an ability to kill all the three tumor cell lines, and, in particular, exhibited a remarkable ability to kill the H1975 strain and the HCC827 strain (Figures 4 to 9).

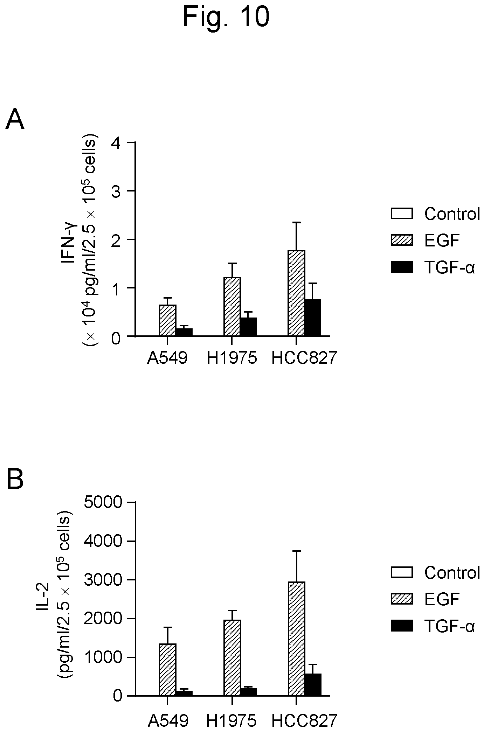

The results of the measurement of cytokine concentrations when T cells and tumor cells were cocultured at an E:T of 1:1 are shown in Figure 10. While the control T cells secreted neither IFN-y nor IL-2, the EGF-type and TGF-α-type CAR-T cells secreted IFN-y and IL-2. This indicates that the EGF-type and TGF-α-type CAR-T cells are capable of killing tumor cells by inducing a cellular immune response through secretion of IFN-y and IL-2 on recognizing an EGFR-expressing tumor cell and becoming activated.

### [Example 4 In vivo anti-tumor cell activities of EGF- and TGF-α-type CAR-T cells]

To evaluate the *in vivo* anti-tumor cell activities of the EGF-type CAR-T cells and TGF-α-type CAR-T cells produced in Example 2, the following experiment was carried out.

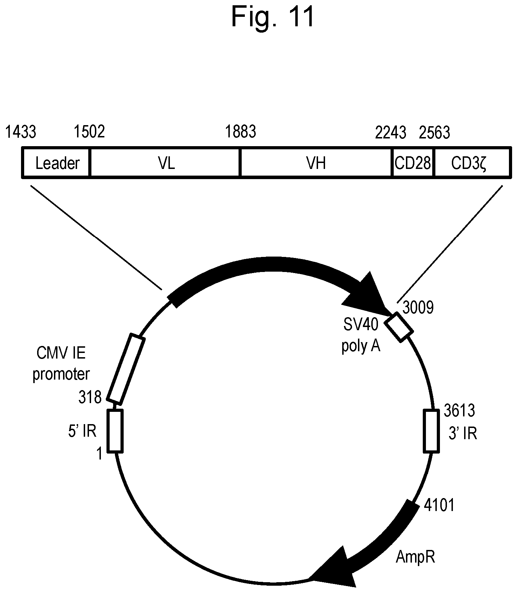

To immunodeficient mice (NOD.Cg-Prkdc^{scid}Il2rg^{tm1Wjl}/SzJ; Charles River Laboratories Japan, Inc.), 2.5 × 10⁶ cells of the non-small cell lung cancer cells HCC827 expressing luciferase (HCC-827-Luc; the National Institutes of Biomedical Innovation, Health, and Nutrition JCRB Cell Bank) were subcutaneously injected, and 7 days after that any one (2.0 × 10⁶ CAR-positive cells) of CD19 CAR-T cells, EGF-type CAR-T cells, and TGF-α-type CAR-T cells was intravenously injected. The EGF-type CAR-T cells and TGF-α-type CAR-T cells used were cell populations cultured until Day 10 in Example 2. The CD19 CAR-T cells used were cell populations obtained by introducing a plasmid encoding CAR comprising an anti-CD19 antibody as an antigen-binding domain (CD19 CAR expression plasmid, SEQ ID NO: 12, Figure 11), stimulating with immature dendritic cells, and culturing until Day 10 in the same manner as in Example 2.

On the day before the T cell injection, and 8, 15, 22, 29, 36, 43, 50, and 63 days after the T cell injection, D-Luciferin (OZ Biosciences USA Inc., San Diego, CA) was intraperitoneally administered to the mice, and 10 minutes after each the residual tumor volume in each mouse was evaluated by measuring bioluminescence with an IVIS lumina II *in vivo* imaging system (PerkinElmer, Inc.). The blood was collected from each mouse 28 days and 63 days after the T cell injection, and the proportion of T cells (the proportion of human T cells to all leukocytes) in the blood was determined.

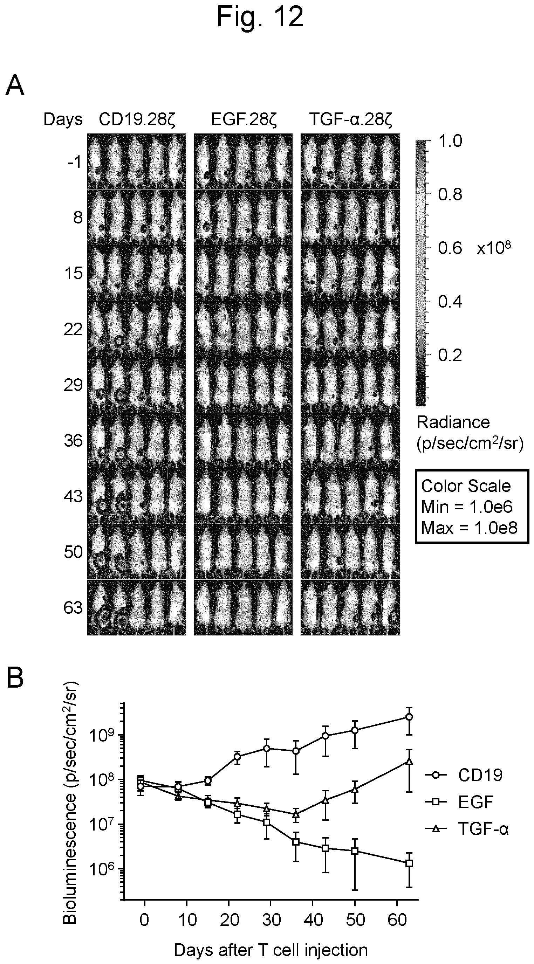

The results of the measurement of bioluminescence are shown in Figure 12. The bioluminescence in the EGF-type CAR-T cells-administered group and that in the TGF-α-type CAR-T cells-administered group were lower than that in the CD19 CAR-T cells-administered group, and thus the antitumor effects of the EGF-type CAR-T cells and TGF-α-type CAR-T cells were found even in the animal experiment. In particular, a remarkable antitumor effect was found in the EGF-type CAR-T cells-administered group.

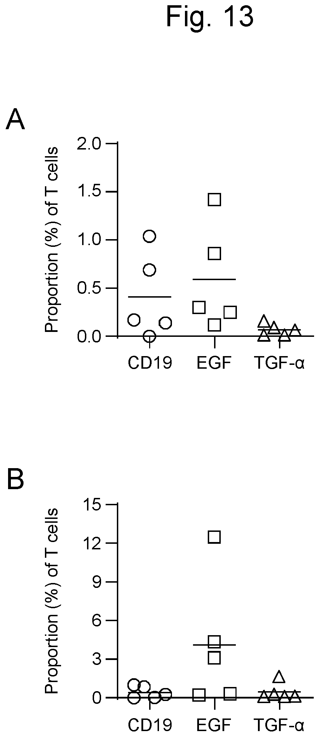

The results of the determination of proportions of T cells are shown in Figure 13. In the EGF-type CAR-T cells-administered group, more T cells remained even after 60 days or more from T cell administration than in the CD19 CAR-T cells-administered group and the TGF-α-type CAR-T cells-administered group. This result indicates that the EGF-type CAR-T cells recognize tumor cells in living bodies and become activated, proliferate, and survive at a high level for a long period of time, and that thereby they exert long-term antitumor effects.

### [Example 5 Production of PVAQ.EGF- and PVAQ.TGF-α-type CAR expression plasmids]

The spacer moiety in the EGF-type CAR and TGF-α-type CAR in Example 1 contains the CH2 region and CH3 region of IgG1. To prevent CAR-T cells from being taken into the lung (captured by alveolar macrophages) through binding of the spacer moiety to Fcy receptor, CARs obtained by introducing the following substitutions into the spacer moiety in the EGF-type CAR and TGF-α-type CAR in Example 1 were designed (PVAQ.EGF-type CAR and PVAQ.TGF-α-type CAR, respectively).
Substitution of ELLG at positions 6 to 9 of CH2 region (SEQ ID NO: 6) in spacer with PVA
Substitution of N at position 70 of CH2 region (SEQ ID NO: 6) in spacer with Q

Mutations to cause the amino acid substitutions were introduced into the EGF-type CAR expression plasmid and TGF-α-type CAR expression plasmid produced in Example 1 to produce a PVAQ.EGF-type CAR expression plasmid and a PVAQ.TGF-α-type CAR expression plasmid.

The nucleotide sequence of the PVAQ.EGF-type CAR expression plasmid is set forth in SEQ ID NO: 16, and the nucleotide sequence of the PVAQ.TGF-α-type CAR expression plasmid is set forth in SEQ ID NO: 17. The amino acid sequence of the spacer in the PVAQ.EGF-type CAR and the PVAQ.TGF-α-type CAR is set forth in SEQ ID NO: 15.

### [Example 6 Culture and amplification of PVAQ.EGF- and PVAQ.TGF-α-type CAR-T cells]

Using the CAR expression plasmids produced in Example 5, CAR-T cells expressing PVAQ.EGF-type CAR or PVAQ.TGF-α-type CAR were produced, and cultured and amplified.

### <6-1 Isolation of PBMCs (Day 0)>

PBMCs were isolated from the peripheral blood of a healthy volunteer donor using Ficoll-PaquePlus.

### <6-2 CAR gene introduction and culture (after Day 0)>

To 2 × 10⁷ cells of the PBMCs isolated in 6-1 above, 7.5 µg of the CAR expression plasmid produced in Example 5 (PVAQ.EGF-type CAR expression plasmid or PVAQ.TGF-α-type CAR expression plasmid), 7.5 µg of a pCMV-piggyBac plasmid, and 100 µl of P3 Primary Cell Nucleofector^{™} solution with addition of Supplement 1 were added, and electroporation was performed with the apparatus 4D-Nucleofector (program FI-115) (Day 0).

The electroporated PBMCs were suspended in ALyS^{™} 705 culture solution containing 10 ng/mL human IL-7, 5 ng/mL human IL-15, and 5% artificial serum, seeded in a 24-well plate, and cultured at 37°C and 5% CO₂.

### <6-3 Production of artificial feeder cells (after Day 0)>

A plasmid for overexpression of truncated EGFR, CD80, and 4-1BBL (pIRII-tEGFR-CD80-4-1-BBL) was electroporated into 2 × 10⁷ cells of the PBMCs isolated in 6-1 above (Day 0). The pIRII-tEGFR-CD80-4-1-BBL was produced by substituting the sequence of truncated HER2 (tHER2) in a pIRII-tHER2-CD80-4-1-BBL plasmid described in Nakamura, K. et al., Mol. Ther. Methods Clin. Dev., 2021, 21, 315-324 with the sequence of truncated EGFR (tEGFR; EGFR truncated into its extracellular domain only). The amino acid sequences of tEGFR, CD80, and 4-1-BBL are set forth in SEQ ID NOs: 18 to 20. The electroporation was performed using P3 Primary Cell Nucleofector^{™} solution and the apparatus 4D-Nucleofector (program FI-115).

Thereafter, the cells were suspended in ALyS^{™} 705 culture solution containing 10 ng/mL human IL-7, 5 ng/mL human IL-15, and 5% artificial serum, and cultured at 37°C and 5% CO₂.

### <6-4 Stimulation and culture of transgenic cells with artificial feeder cells>

The cells cultured for 18 hours in 6-3 above were UV-irradiated, and then added to the CAR gene-introduced cells cultured in 6-2 above, and culture was continued until any of Days 12 to 14. Half-volume of the culture solution was exchanged every 2 to 3 days.

### <6-5 Evaluation of CAR expression rates>

On Day 14, the CAR expression rates in the cell populations having the PVAQ.EGF-type CAR expression plasmid introduced thereinto were evaluated according to the method described in Example 2. As a result, the CAR expression rates were found to be more than 30%.

### [Example 7 In vivo anti-tumor cell activities of PVAQ.EGF- and PVAQ.TGF-α-type CAR-T cells]

To evaluate the *in vivo* anti-tumor cell activities of the PVAQ.EGF-type CAR-T cells and PVAQ.TGF-α-type CAR-T cells produced in Example 6, the following experiment was carried out.

To immunodeficient mice (NOD.Cg-Prkdc^{scid}Il2rg^{tm1Wj1}/SzJ; Charles River Laboratories Japan, Inc.), 3.0 × 10⁶ cells of the non-small cell lung cancer cells H1975 expressing luciferase (NCI-H1975-Luc; the National Institutes of Biomedical Innovation, Health, and Nutrition JCRB Cell Bank) were subcutaneously injected, and 7 days after that any one (2.0 × 10⁶ CAR-positive cells) of CD19 CAR-T cells, PVAQ.EGF-type CAR-T cells, and PVAQ.TGF-α-type CAR-T cells was intravenously injected. The PVAQ.EGF-type CAR-T cells and PVAQ.TGF-α-type CAR-T cells used were cell populations cultured until Day 14 in Example 6. The CD19 CAR-T cells used were cell populations obtained by introducing a CD19 CAR expression plasmid (SEQ ID NO: 12, Figure 11) into PBMCs, stimulating with artificial feeder cells, and culturing until Day 14 in the same manner as in Example 6.

From the day of T cell injection until 63 days after the T cell injection, D-Luciferin (OZ Biosciences USA Inc., San Diego, CA) was intraperitoneally administered to the mice once per week, and 10 minutes after each the residual tumor volume in each mouse was evaluated by measuring bioluminescence with an IVIS lumina II *in vivo* imaging system (PerkinElmer, Inc.). The blood was collected from each mouse 24 days after the T cell injection, and the proportion of T cells (the proportion of human T cells to all leukocytes) in the blood was determined. Each mouse was euthanized when the mouse exhibited a clear sign of discomfort or at the end of the experiment.

The results of the measurement of bioluminescence are shown in Figure 14A. The PVAQ.EGF-type CAR-T cells and PVAQ.TGF-α-type CAR-T cells exhibited higher antitumor effects than the CD19 CAR-T cells. In particular, a remarkable antitumor effect was found in the mice to which the PVAQ.EGF-type CAR-T cells had been administered.

The results of measurement of survival rates are shown in Figure 14B. The mice to which the PVAQ.EGF-type CAR-T cells or the PVAQ.TGF-α-type CAR-T cells had been administered exhibited higher survival rates than the mice to which the CD19 CAR-T cells had been administered. In particular, a remarkably enhanced survival rate was found for the mice to which the PVAQ.EGF-type CAR-T cells had been administered. As a result of log-rank test, the PVAQ.EGF-type CAR-T cells-administered group and the PVAQ.TGF-α-type CAR-T cells-administered group were found to have significantly prolonged survival time as compared to the CD19 CAR-T cells-administered group (p < 0.01). In addition, the PVAQ.EGF-type CAR-T cells-administered group was found to have significantly prolonged survival time as compared to the PVAQ.TGF-α-type CAR-T cells-administered group (p < 0.05).

The results of the determination of proportions of T cells in blood are shown in Figure 14C. In the PVAQ.EGF-type CAR-T cells-administered group, more T cells remained even 24 days after the T cell administration than in the CD19 CAR-T cells-administered group and the PVAQ.TGF-α-type CAR-T cells-administered group. This result was consistent with that in Example 4.

In the present experiment, the mice did not die without the growth of tumor. The mice each kept appetite and were active with normal hair and normal appearance from the injection with CAR-T cells until death due to tumor or until being euthanized.

### [Example 8 In vitro anti-tumor cell activity of EGF-type CAR-T cells]

Coculture test was carried out with EGF-type CAR-T cells and tumor cells, using tumor cell lines differing from those in Example 3, to further evaluate the anti-tumor cell activity of the EGF-type CAR-T cells.

The coculture test was carried out according to the method described in Example 3. EGF-type CAR-T cells and control T cells (T cells activated with an anti-CD3 antibody and an anti-CD28 antibody) were produced according to the method described in that Example. The non-small cell lung cancer cell line H460 or RERF-LC-Sq1 (NCI-H460-Luc, RERF-LC-Sq1/CMV-Luc; the National Institutes of Biomedical Innovation, Health, and Nutrition JCRB Cell Bank) was used as tumor cells.

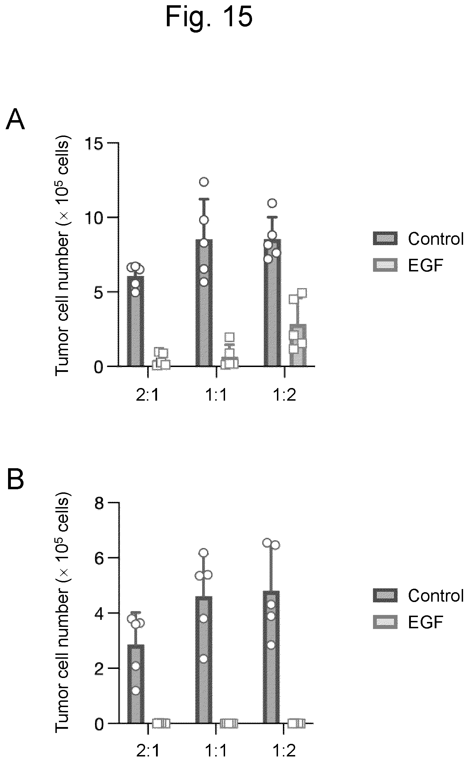

The results of measurement of tumor cell numbers are shown in Figure 15. The EGF-type CAR-T cells caused more reduction in tumor cell numbers than the control T cells did at all of the E:T ratios tested.

Non-small cell lung cancer is primarily classified into the following three tissue types: adenocarcinoma, squamous cell cancer, and large cell cancer. The tissue types of the non-small cell lung cancer cell lines H460 and RERF-LC-Sq1 used in this Example were large cell cancer and squamous cell cancer, respectively, and the tissue types of the non-small cell lung cancer cell lines A549, H1975, and HCC827 used in Examples 3, 4, and 7 were each adenocarcinoma.

From the results of this Example and Examples 3, 4, and 7, in which the EGF-type CAR-T cells exhibited antitumor effects to all the above non-small cell lung cancer cell lines, the CAR-T cells of the present invention were demonstrated to exert antitumor effects irrespective of tissue type.

### Sequences

SEQ ID NO: 1: nucleotide sequence encoding EGF protein 
AATAGTGACTCTGAATGTCCCCTGTCCCACGATGGGTACTGCCTCCATGATGGTGT
GTGCATGTATATTGAAGCATTGGACAAGTATGCATGCAACTGTGTTGTTGGCTAC
ATCGGGGAGCGATGTCAGTACCGAGACCTGAAGTGGTGGGAACTGCGC
SEQ ID NO: 2: amino acid sequence of EGF protein
   NSDSECPLSHDGYCLHDGVCMYIEALDKYACNCVVGYIGERCQYRDLKWWELR
SEQ ID NO: 3: nucleotide sequence encoding TGF-α protein 
GTGGTGTCCCATTTTAATGACTGCCCAGATTCCCACACTCAGTTCTGCTTCCATGG
AACCTGCAGGTTTTTGGTGCAGGAGGACAAGCCAGCATGTGTCTGCCATTCTGGG
TACGTTGGTGCACGCTGTGAGCATGCGGACCTCCTGGCC
SEQ ID NO: 4: amino acid sequence of TGF-α protein
   VVSHFNDCPDSHTQFCFHGTCRFLVQEDKPACVCHSGYVGARCEHADLLA
SEQ ID NO: 5: amino acid sequence of hinge region of IgG1
   EPKSCDKTHTCPPCDPAEPKSPDKTHTCP
SEQ ID NO: 6: amino acid sequence of CH2 region of IgG1 
PCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAK
SEQ ID NO: 7: amino acid sequence of CH3 region of IgG1 
GQPREPQVYTLPPSRDELTKNQVSLTCLVKGFYPSDIAVEWESNGQPENNYKTTPPVL
DSDGSFFLYSKLTVDKSRWQQGNVFSCSVMHEALHNHYTQKSLSLSPGK
SEQ ID NO: 8: amino acid sequence of human CD28-derived transmembrane domain and costimulatory domain 
FWVLVVVGGVLACYSLLVTVAFIIFWVRSKRSRLLHSDYMNMTPRRPGPTRKHYQP
YAPPRDFAAYRS
SEQ ID NO: 9: amino acid sequence of human CD3ζ chain-derived intracellular signaling domain 
RVKFSRSADAPAYQQGQNQLYNELNLGRREEYDVLDKRRGRDPEMGGKPRRKNPQ
EGLYNELQKDKMAEAYSEIGMKGERRRGKGHDGLYQGLSTATKDTYDALHMQALP
PR
SEQ ID NO: 10: EGF-type CAR expression plasmid
SEQ ID NO: 11: TGF-α-type CAR expression plasmid
SEQ ID NO: 12: CD19 CAR expression plasmid
SEQ ID NO: 13: leader sequence
MEFGLSWLFLVAILKGVQCSR
SEQ ID NO: 14: amino acid sequence of spacer 
EPKSPDKTHTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSLSLSP
GKKDPK
SEQ ID NO: 15: amino acid sequence of modified spacer 
EPKSPDKTHTCPPCPAPPVAGPSVFLFPPKPKDTLMISRTPEVTCVVVDVSHEDPEVKF
NWYVDGVEVHNAKTKPREEQYQSTYRVVSVLTVLHQDWLNGKEYKCKVSNKALPA
PIEKTISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSLSLSPG
KKDPK
SEQ ID NO: 16: nucleotide sequence of PVAQ.EGF-type CAR expression plasmid
SEQ ID NO: 17: nucleotide sequence of PVAQ.TGF-α-type CAR expression plasmid
SEQ ID NO: 18: amino acid sequence of tEGFR
SEQ ID NO: 19: amino acid sequence of CD80
SEQ ID NO: 20: amino acid sequence of 4-1-BBL

All publications, patents and patent applications cited herein are incorporated herein by reference in their entirety.

## Claims

1. A polynucleotide encoding a chimeric antigen receptor (CAR) protein having a target-binding domain that binds to epidermal growth factor receptor (EGFR), a transmembrane domain, and an intracellular signaling domain, wherein the target-binding domain is a ligand for EGFR.

2. The polynucleotide according to claim 1, wherein the target-binding domain is epidermal growth factor (EGF) or transforming growth factor-α (TGF-α).

3. The polynucleotide according to claim 1, wherein the target-binding domain is: a polypeptide consisting of an amino acid sequence set forth in SEQ ID NO: 2 or 4; or a polypeptide that comprises an amino acid sequence having a sequence identity of at least 90% with an amino acid sequence set forth in SEQ ID NO: 2 or 4 and has an ability to bind to EGFR.

4. A vector comprising the polynucleotide according to claim 1.

5. A genetically modified cell having the polynucleotide according to claim 1 or the vector according to claim 4 introduced thereinto.

6. A method for producing a CAR protein-expressing cell, comprising introducing the polynucleotide according to claim 1 or the vector according to claim 4 into a cell.

7. A therapeutic agent for a disease involving an EGFR-expressing cell, comprising the cell according to claim 5.

8. A pharmaceutical composition comprising the therapeutic agent according to claim 7 and a pharmaceutically acceptable carrier.

9. The therapeutic agent according to claim 7, wherein the disease involving an EGFR-expressing cell is selected from lung cancer, esophageal cancer, gastric cancer, pancreatic cancer, biliary tract cancer, colon cancer, rectal cancer, head and neck cancer, glioblastoma, brain cancer, breast cancer, ovarian cancer, squamous cell cancer, and adenocarcinoma.

10. A kit for producing a CAR protein-expressing cell targeting an EGFR-expressing cell, wherein the kit comprises the vector according to claim 4.
